(19) Europäisches Patentamt
European Patent Office
Office européen des brevets

(11) **EP 2 450 062 A1**

(12) **EUROPEAN PATENT APPLICATION**
published in accordance with Art. 153(4) EPC

(43) Date of publication:
**09.05.2012 Bulletin 2012/19**

(51) Int Cl.:
***A61L 15/36*** *(2006.01)*      ***A61K 35/74*** *(2006.01)*

(21) Application number: **10793498.6**

(22) Date of filing: **01.07.2010**

(86) International application number:
**PCT/CL2010/000025**

(87) International publication number:
**WO 2011/000123 (06.01.2011 Gazette 2011/01)**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **01.07.2009 CL 15062009**

(71) Applicant: **Universidad De Concepcion Santiago (CL)**

(72) Inventors:
 • **CASTRO INOSTROZA, Erica**
   **Concepción (CL)**

 • **BORQUEZ YAÑEZ, Rodrigo**
   **Concepción (CL)**
 • **GONZALES RIQUELME, Margarita**
   **Concepción (CL)**
 • **KLATTENHOFF STOHR, Dietter**
   **Concepción (CL)**

(74) Representative: **Carvajal y Urquijo, Isabel et al**
   **Clarke, Modet & Co.**
   **Patentes**
   **C/Goya no. 11**
   **28001 Madrid (ES)**

(54) **HYDROCOLLOID PLASTERS, INCLUDING VIABLE PROBIOTIC STRAINS OF LACTOBACILLUS SPP. AND USED FOR VARIOUS TYPES OF WOUNDS, INFECTED TISSUES, DRY WOUNDS AND DEEP WOUNDS**

(57)    This invention involves a formulation comprising a plaster or dressing based on a hydrocolloid that is a natural gelatin, and viable lactic acid-producing probiotic strains of *Lactobacillus spp.,* preferably the strain *Lactobacillus acidophilus LPV 31,* as the active principle in the treatment of various types of wounds, mainly ulcers, burns (up to 2nd degree burns), and/or surgical wounds, as well as infected tissue, dry wounds and deep wounds.

EP 2 450 062 A1

**Description**

**Field of the Invention**

This technology is oriented towards clinical use, specifically the treatment of patients with varying degrees of burns and different types of skin wounds. This invention involves a probiotic formulation based on hydrocolloid plasters and viable lactic acid-producing probiotic strains.

**State of the Art**

In the United States, 2 million accidents occur each year that cause major or minor burns. While most cases are minor, approximately 70,000 require hospitalisation, and 20,000 are treated in special burn care units. In Chile, at least 3,000 people each year suffer burns that can lead to or be associated with various infections.

For many years, antibiotics have been used to prevent infections in wounds such as burns. The basis for this practice is the notion that burn patients, having suffered damage to the skin, the main external defence barrier of the body, as well as having a compromised immune system, are defenceless against various infection-causing microorganisms which then must be treated. The use of a specific antibiotic, however, only eliminates the flora sensitive to it, leading to an overpopulation of resistant bacteria which must then be treated with other -possibly more expensive and more toxic- antibiotics that can create conditions for new infections by more aggressive bacteria or fungi, therefore creating an overall larger public health problem.

Currently, there is a large number of wound care products at our disposal, and various materials have been developed to improve wound healing in a moist environment. The wide array of wound care products related to this type of healing can be classified in different ways according to the properties analysed:

i. *Polyurethane films:* synthetic films, permeable to water vapour, oxygen and other gases and impermeable to water and bacteria. They are usually transparent, with low absorbency, and therefore only indicated in wounds with low exudate, such as chronic ulcers. in the process of scarring, donor areas, superficial wounds, excoriations and lacerations, first and second-degree burns, surgical and cut wounds, and to protect vascular accesses and catheter entry sites.

ii. *Polymeric foam dressings, developed through the modification of polyurethane foam:* dressings with good absorbency that integrate part of the exudate into their structure, with the rest of the exudate evaporating due to its water vapour permeability.

iii. *Hydrogel dressings:* dressings with higher absorbency; they eliminate toxic components from the wound bed and maintain the moisture level and temperature in the wound area. As hydrogel is not adhesive, a secondary dressing is necessary to hold it in place.

iv. *Alginates (belonging to the group phycocolloids):* composed of alginic acid salts, a polymer that is an essential component of the cell wall of brown algae and that is extracted for its pharmacological use, alginates have a very high absorbent capacity of up to 20 times their weight. They also promote debridement as, when in contact with the exudate, a gradual ion-exchange process transforms the (insoluble) calcium alginate into (soluble) sodium alginate, thus providing the wound bed with a moist, intact surface. This creates optimal healing conditions, the gel being rich in natural proteins, amino acids and growth factors produced naturally in the wound bed.

Knowledge of the biological process of wound healing is essential, as an efficient treatment is one that does not interfere with the natural healing process, i.e. the tendency to recover as it is assisted in its successive stages. The problem has been how to treat wounds properly in order to accelerate healing, as rapid healing reduces complications and discomfort to the patient. This has been especially critical in the past few years, due to a huge increase in new drugs and procedures that assist the healing process.

In recent years, new technologies and materials have revolutionised the treatment of wounds, ulcers and burns. New technologies have also been developed for the controlled and sustained release of medicine within a specific time lapse, contributing to a reduction in adverse effects and the achievement of the desired therapeutic effect. The use of these products, however, is contraindicated in patients with a known sensitivity to the dressing or its components, as well as with infected wounds, serious burns, complex wounds and chronic ulcers.

These problems and contraindications will be solved thanks to our innovative technology.

Various documents exist that describe inventions that could be considered relevant to this initiative. Following is a discussion of the most relevant documents within this category:

World patent application WO2008074331 protects a dressing for wounds or damaged tissue that consists of an absorbent component, an amorphous hydrogel to absorb the wound exudate, and a lyophilised and encapsulated bacterium that is able to produce lactic acid from sugars or from the fermentation of sugars, the said bacteria belonging to the lactic acid bacteria family, selected from the groups *Camobacterium, Enterococcus, Laxtobacillus,* etc. The application of this dressing in treating individuals with wounds and damaged tissue is also protected. Despite the fact that this technology contains lactic acid-producing bacteria, these are lyophilised and encapsulated, a significant difference with regard to the technology we seek to protect.

World patent application WO2008060199 to the World Intellectual Property Organization describes a hygiene tissue, i.e. wet wipe, dry wipe, plaster, napkin, etc. with a microbe-inhibiting composition comprising an extracellular product of at least one lactic acid-producing bacterium, such as *Lactobacillus plantarum* LB931 or *Lactobacillus fermentu* Ess-1 or a combination thereof, and at least one additive in the form of an organic acid, with a minimum of one of its pKa value not exceeding 5.5, and/or a salt thereof. These species are not used in our invention and, moreover, it is not the species themselves but an extracellular product of these species that is used. The novelty of our technology, therefore, is not affected.

American invention patent US2002131996 protects a method for blocking microbial adherence to the surface of eukaryotic cells, as well as a pharmacological composition that consists of isoleucine and live probiotic microorganisms such as lactobacilli. This composition can be presented in different pharmaceutical forms, including that of a food product. The main component of this invention is isoleucine together with the added probiotic microorganisms. In our technology the main product is the probiotic and the formula does not contain isoleucine. Both are clear differences from the aforementioned product.

Invention patent WO0071139 of the World Intellectual Property Organization protects a probiotic composition and its by-products, as well as the method used in these compositions to inhibit, treat or prevent infections in wounds, in addition to promoting biofilm formation on surgical instruments. This method consists in administering a therapeutically effective number of probiotic organisms *(Lactobacillus sp.)* to the affected area, thereby stimulating immune response against an infection and inhibiting pathogen colonisation in the host. Its pharmaceutical composition comprises an effective number of prebiotic and probiotic organisms *(Lactobacillus sp.)* and collagen for protein union. The description of this patent does not interfere with our technology, as the methodology is very different from that which we seek to protect.

In accordance with the above information, no previous document exists in the prior art containing the totality of our invention.

## Description of the Invention

This invention involves a formulation based on a hydrocolloid that is a natural gelatin, and the use of viable lactic acid-producing probiotic strains *Lactobacillus spp.,* preferably the strain *Lactobacillus acidophilus LPV 31,* as the active principle in the treatment of various types of wounds, mainly ulcers, burns (up to 2nd degree burns), and/or surgical wounds.

This formulation also incorporates a cross-linking agent and a plasticiser. These components are used to create a natural gelatin-based, reticulated film that is used as a plaster, dressing or bandage, i.e. a vehicle for administering probiotic microorganisms by enabling their survival. This innovative method can surprisingly be used even in infected tissue, dry wounds and deep wounds, which are problematic cases that have not been solved by the conventional technologies previously described.

Natural gelatin, a water-soluble protein, possesses many favourable properties. It is an organic product, obtained naturally, that is biocompatible, biodegradable, non-immunogenic and that can absorb a large quantity of water. Gelatin is used at concentrations ranging from 1-10% w/w, with a preferable concentration of 7% w/w.

In terms of the probiotic bacteria, the group selected is *Lactobacillus spp.,* preferably *Lactobacillus acidophilus* LPV-31, which can be incorporated into the gelatin films, retaining its probiotic characteristics during storage. This strain is used because the biofilm that it forms creates a protective layer that helps to avoid or combat infections. Another important factor is the acid pH that it produces -an extremely useful property in the production of a plastic dressing- as it creates a hostile environment for pathogens and thereby both helps wounds heal and prevents infections.

The cross-linking agent used is preferably, but not exclusively, glutaraldehyde (GA), due to its high efficiency stabilising collagen derivatives, its high reaction rate, and its ability to modify the physico-chemical properties of gelatin-based gels. The formulation contains glutaraldehyde concentrations between approximately 0.0005-0.006 M. These amounts do not have an observable bactericidal effect and therefore similar counts of bacteria are obtained with the different concentrations.

The formulation plasticiser is glycerol, used in a concentration range of approximately 1-10% v/v, preferably 6% v/v. This concentration range makes it possible to obtain gelatin films with greater flexibility that are easier to manipulate when used as a dressing.

The production process for this technology consists of the following stages:

1. Activation of the lactic acid-producing probiotic strains of *Lactobacillus acidophilus* LPV 31.
2. Production of reticulated gelatin films, incorporating viable probiotic strains of *Lactobacillus acidophilus* LPV 31.
3. Drying stage (vacuum or infrared drying).
4. Viability analysis.

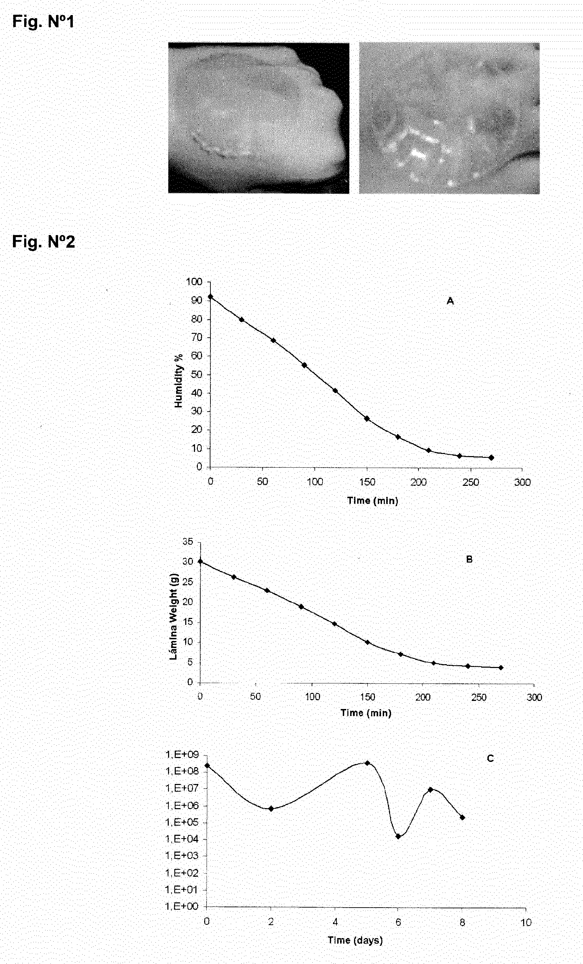

The final product, as shown in **Figure 1,** is a reticulated gelatin film with a diameter of 50 mm and a thickness of 0.5 mm, approximately.

In order to determine specific properties of the technology, a series of tests were carried out, including, among others: drying tests, mechanical tests, viability tests and hydration tests, as well as certain physico-chemical tests, the results of which are presented below:

Drying tests using infrared light were carried out on films without bacteria. **Figure 2A** shows that after approximately 200 minutes, a moisture level of around 10% was achieved, leading to a subsequent loss of weight in the gelatin film, as seen in **2B.** Subsequently, the bacteria were incorporated into the gelatin films and viability was tested. These results are shown in **Figure 2C,** where it can be seen that the strains remained viable and that their concentration in the gelatin film did not vary significantly over time.

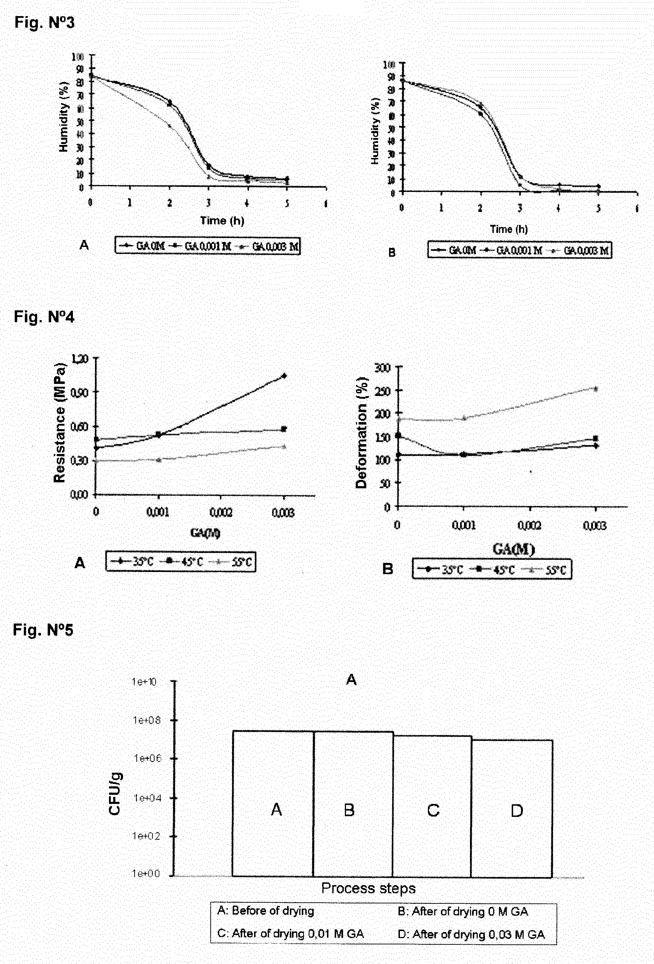

The graphs in **Figure 3** show the effects of temperature, the concentration of the cross-linking agent glutaraldehyde (GA), and that of the plasticiser glycerol (GLI) on the vacuum drying curves of the gelatin films obtained.

At a temperature of 35°C, with a glycerol concentration of 6% and 8%, respectively, the drying time necessary to reach a moisture level under 10% is 5 h. When increasing the temperature to 45°C **(Figures 3A and 3B),** with a glycerol concentration of 6% and 8%, respectively, this time is reduced to 3 h. A similar result is observed when the drying temperature is increased to 55°C, with a glycerol concentration of 6% and 8%, respectively. The optimal temperature for the preparation of the films is considered to be 45°C, as it does not affect the incorporated bacteria in the drying process.

It is possible to obtain moisture levels under 10% in films with both the vacuum and infrared drying methods, and the concentration of glutaraldehyde does not have a significant effect on the drying times of the reticulated films. Furthermore, the GA concentrations studied do not have an observable bactericidal effect, and similar bacterial counts are obtained in each test.

With regard to the mechanical tests, **Figure 4** shows the graphs corresponding to resistance and deformation tests **(Figures 4A and 4B,** respectively) carried out with a glycerol concentration of 6%, for different temperatures and concentrations of glutaraldehyde. Mechanical tests carried out on the films dried to under 10% moisture indicate sufficient resistance and flexibility for their use as a dressing in wound treatment.

Viability tests were carried out to ensure that the strains of probiotic bacteria survive the drying stage applied to the gelatin film. **Figure 5** shows these tests for the drying stage **(Figure 5A)** and for the stored dry product **(Figure 5B).** The method used to determine viability in films stored at 4°C was a bacterial count on plates with MRS agar. Thus it was determined that vacuum drying conditions are in fact compatible with the probiotic strain.

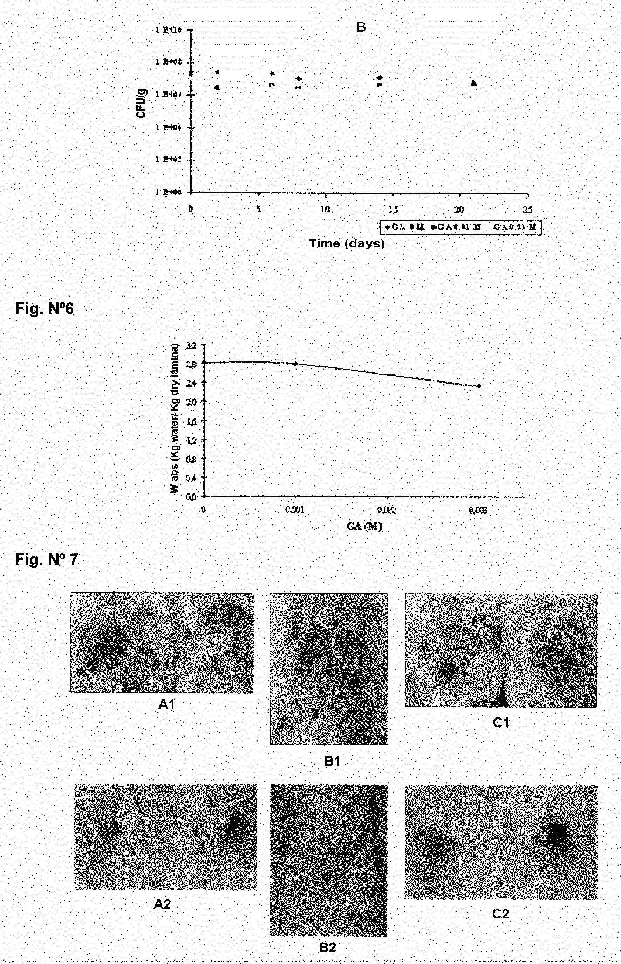

As a way of determining the water absorption capacity of the gel, hydration tests were carried out on dry gelatin films. **Figure 6** shows the graph corresponding to the hydration tests with different concentrations of glutaraldehyde. It is observed that as the GA concentration increases to 0.003 M, hydration capacity is reduced to 2.4 kg water/kg of dry film. Notwithstanding, this effect is only evident for GA concentrations higher than 0.001 M. This indicates that the gel films manufactured using this technology will have a significant exudate absorption capacity when applied to wounds.

In addition to physical tests, a series of tests were carried out to determine whether the said films are effectively an efficient vehicle for administering live microorganisms, as well as whether they retain their characteristics after drying and storage.

**Table 1** shows the films with different concentrations of glutaraldehyde (M) used to determine probiotic properties through biochemical tests. A portion of the films subjected to these tests underwent storage for one week and others were dried the same day. Letters A and B correspond to the concentration of glycerol used: 6% and 8%, respectively.

**Table 1**

| Films stored for one week | | Films dried on the same day | |
|---|---|---|---|
| 1.1 A | 0 | 1.2 A | 0 |
| 1.1 B | 0 | 1.2 B | 0 |
| 2.1 A | 0.001 | 2.2 A | 0.001 |
| 2.1 B | 0.001 | 2.2 B | 0.001 |

(continued)

| Films stored for one week | | Films dried on the same day | |
|---|---|---|---|
| 3.1 A | 0.003 | 3.2 A | 0.003 |
| 3.1 B | 0.003 | 3.2 B | 0.003 |

**Table 2** shows a series of biochemical tests to determine probiotic properties during the process. It can be observed that the strains contained in both the films that were stored for one week and those that were dried the same day conserve their properties, irrespective of different concentrations of glutaraldehyde and glycerol. These tests confirm the feasibility of incorporating *Lactobacillus acidophilus* LPV 31 into the gelatin films, as the probiotic properties of the strain are preserved during storage.

**Table 2**

| Sample | CO2 Production | | Nitrate | NH3 Production Arginine | Indole Reduction | Esculin Hydroysis | Bacterial Development | |
|---|---|---|---|---|---|---|---|---|
| | Gibson | Gluconate | | | | | MRS 15- C | MRS 45- C |
| LPV 31 | - | - | - | - | - | + | + | + |
| 1.1 A | - | - | - | - | - | + | + | + |
| 1.1 B | - | - | - | - | - | + | + | + |
| 2.1 A | - | - | - | - | - | + | + | + |
| 2.1 B | - | - | - | - | - | + | + | + |
| 3.1 A | - | - | - | - | - | + | - | + |
| 3.1 B | - | - | - | - | - | + | + | + |
| 1.2 A | - | - | - | - | - | + | + | + |
| 1.2 B | - | - | - | - | - | + | + | + |
| 2.2 | - | - | - | - | - | + | - | + |
| 3.2 | - | - | - | - | - | + | + | + |

The results of the hydrophobicity tests can be seen in Table 3. These were obtained using a partitioning technique in organic solvents such as hexadecane, toluene and xylene, with hydrophobicity being determined by the tendency of the bacteria to move from the aqueous phase to the organic phase.

**Table 3**

| Sample | Hexadecane | Toluene | Xylene |
|---|---|---|---|
| LPV 31 | 94.00 | 91.00 | 81.00 |
| 1.1 A | 79.51 | 87.12 | 91.99 |
| 1.1 B | 92.07 | 77.94 | 94.49 |
| 2.1 A | 83.06 | 100.00 | 90.49 |
| 2.1 B | 95.11 | 93.04 | 85.92 |
| 3.1 A | 75.85 | 81.65 | 81.43 |
| 3.1 B | 87.91 | 100.00 | 94.07 |
| 1.2 A | 94.34 | 100.00 | 99.09 |
| 1.2 B | 92.42 | 83.44 | 96.99 |
| 2.2 | 100.00 | 98.80 | 100.00 |
| 3.2 | 90.78 | 84.11 | 88.22 |

**Table 4** shows that hydrogen peroxide production was not affected by the drying process. This shows that the strain's capacity to combat pathogens through the production of peroxide is not altered by the drying process, or by storage conditions.

**Table 4**

| Sample | Peroxide |
|--------|----------|
| LPV 31 | + |
| 1.1 A | + |
| 1.1 B | + |
| 2.1 A | + |
| 2.1 B | + |
| 3.1 A | + |
| 3.1 B | + |
| 1.2 A | + |
| 1.2 B | + |
| 2.2 | + |
| 3.2 | + |

This innovative technology was validated through a test carried out on mice, using reticulated gelatin plasters containing the strain *Lactobacillus acidophilus* LPV 31. A burn wound was produced on the back of the animals and the dressing was applied, after which the evolution of the wound was observed over time. The wound evolution results are shown in **Figure 7.**

In order to evaluate different parameters, another test was carried out in which mice were infected with the pathogenic strain *Pseudomonas aeruginosa* P65-00. These were subsequently treated with reticulated gelatin plasters with and without strains of *Lactobacillus acidophilus* LPV 31.

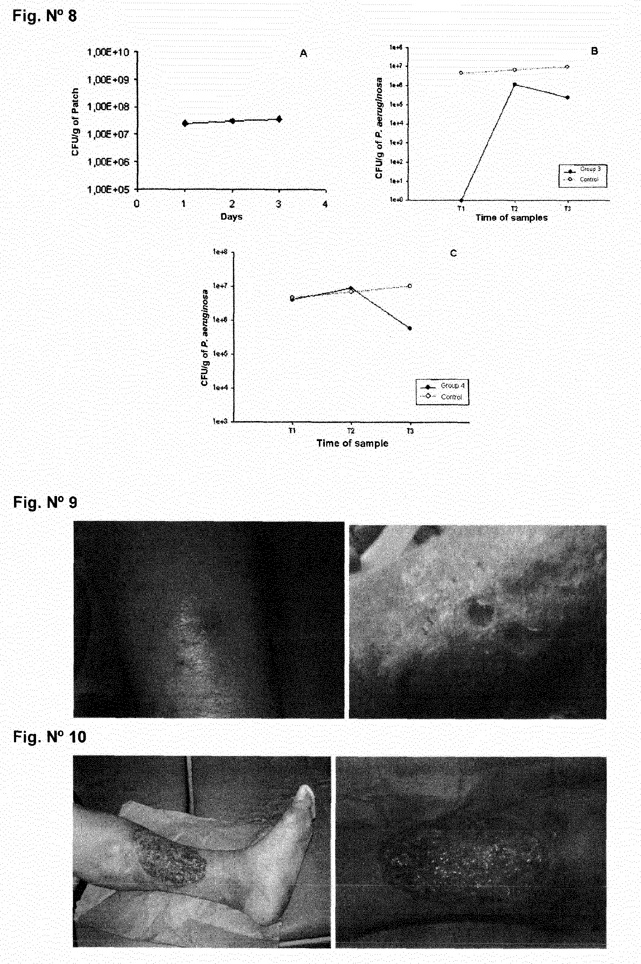

The viability averages of the plasters show a concentration of bacteria that remains constant over the time in which they were applied (i.e. one week), as shown in **Figure 8A.**

The results presented in **Table 5** show that only the pathogenic strain was able to remain in the place of the injury, maintaining high concentrations until the end of the experiment. On the other hand, it was only possible to isolate the probiotic strain in the , first two samples and in low concentrations. The bacterial count of the groups in the wound area was obtained in CFU/g. The said groups were divided into:

- Group 1: Plaster (negative control, **P)**
- Group 2: Strain LPV 31 incorporated into a plaster **(P+31)**
- Group 3: Strain LPV 31 incorporated into a plaster and strain Ps65 **(P+31+Ps)**
- Group 4: Ps65 and strain LPV 31 incorporated into a plaster **(Ps+P+31)**
- Group 5: Ps65 (positive control, **Ps)**
  \*NG No growth

**Table 5**

|  | T1 | | T2 | | T3 | |
|--|------|--------|------|--------|------|--------|
|  | **LPV31** | **P65-00** | **LPV31** | **P65-00** | **LPV31** | **P65-00** |
| **Group 1 (P)** | NG | NG | NG | NG | NG | 6.93E+02 |
| **Group 2 (P+31)** | 1.61E+02 | NG | 6750 | NG | - | - |
| **Group 3 (P+31+Ps)** | 4.30E+02 | NG | NG | 1.19E+06 | NG | 2.43E+05 |
| **Group 4 (Ps+P+31)** | NG | 3.99E+06 | 1147.333333 | 8.67E+06 | NG | 5.59E+05 |
| **Group 5 (Ps)** | NG | 4.62E+06 | NG | 6.81E+06 | NG | 1.02E+07 |

In the haematological tests there were no mice with hematogenous infections, either with the probiotic strain or with the pathogenic strain. Nor was there any contamination with other bacteria.

The count carried out on splenic tissue shows the pathogenic strain PS 65-00 in low concentrations within the first three sampling times and only in some groups, as can be seen in **Table 6.**

**Table 6**

|  | T1 | T2 | T3 |
|---|---|---|---|
| **Group 1 (P)** | NG | NG | NG |
| **Group 2 (P+31)** | NG | NG | - |
| **Group 3 (P+31+Ps)** | NG | 1.63E+01 | 33.95 |
| **Group 4 (Ps+P+31)** | 1.10E+01 | 8.15 | NG |
| **Group 5 (Ps)** | 6.73E+01 | 1.16E+01 | NG |

Bacterial count in the hepatic tissue, presented in **Table 7,** indicates the presence of pathogenic strain PS 65-00 within the first three sampling times and only in some groups, similar to the results for splenic tissue. Concentrations were also low, with the exception of the preventive group, group 3 (P+31+Ps), which contained a high concentration of the pathogenic strain.

**Table 7**

|  | T1 | T2 | T3 |
|---|---|---|---|
| **Group 1 (P)** | NG | NG | NG |
| **Group 2 (P+31)** | NG | NG | - |
| **Group 3 (P+31+Ps)** | NG | 6.50E+06 | 3.435 |
| **Group 4 (Ps+P+31)** | 4.00E+00 | 27,500 | NG |
| **Group 5 (Ps)** | 4.30E+00 | NG | NG |

The preventive effect over time of probiotic strain LPV31 on the intrahospital strain P65-00 in the wound area can be observed in Figure 8B, where group 3 represents the preventive role (•) plaster+LPV31, with the subsequent inoculation of pathogenic strain P65-00. The control (□) only consists of inoculation with P *aeruginosa* P65-00.

The healing effect of probiotic strain LPV31 with regard to intrahospital strain P65-00 in the wound area as a function of time can be observed in Figure 8C. Group 4 corresponds to the healing factor, with the introduction of pathogenic strain PS65-00 and subsequent application of the plaster+LPV 31. The control only consists of inoculation with P *aeruginosa* P65-00.

The data obtained from these tests shows that the dressing that contains the probiotic strain can inhibit the effect of the pathogenic bacteria, playing a role that is both preventive and curative.

Finally, this innovative technology was tested by applying it to patients with severe burns and chronic ulcers, obtaining surprising results that confirm a clear inhibition of pathogenic microorganism growth in the wound area.

## APPLICATION EXAMPLES

### Example 1: Strain activation

The strain *Lactobacillus acidophilus* LPV 31 was activated for 48 hours before inoculating the bacteria into the gelatin solution. This procedure is described below:

a) The strain was activated, adding 100 $\mu$l to a tube with 4 ml of culture medium (MRS), and incubated in a stove at 37°C for 24 h.

b) After this period 100 $\mu$l were transferred to each one of the 8 tubes with 4 ml of culture medium (MRS) and incubated in a stove at 37°C for 12 hours to ensure a biomass larger than $10^8$ CFU.

c) 4 volumetric flasks with 400 ml of whey permeate were inoculated with 700 $\mu$l each, with an optical density between 5 and 6, and left for 14 h in a stove at 37°C at 90 rpm shaking frequency.

d) 14 hours after the strain was activated, the volumetric flasks were removed from the stove and transferred to centrifuge tubes. The tubes were centrifuged at 3,500 rpm for 11 minutes, after which the supernatant was discarded, leaving approximately 18 ml of pellet.

e) Subsequently, 1 ml of pellet was added to the vial with the gelatin solution, taken out of the thermostatic bath 10 minutes beforehand, and mixed with a vortex mixer.

**Example 2: Production process of the reticulated gelatin film**

This process consisted of the following stages:

a) An amount of gelatin at 98%, previously weighed (depending on the weight percentage of gelatin to be prepared), was placed in a vial with 4 ml of distilled water and shaken until the gelatin dissolved. Once dissolved in distilled water at the required concentration, it was then autoclaved.

b) Sterile glutaraldehyde in a concentration of 0.003 M was added to the vials with the autoclaved mix of 5 ml of gelatin at 7% w/w and glycerol at 6%. The vials were covered and put in a thermostatic bath at 55°C for 30 min for the glutaraldehyde to react with the gelatin.

c) Once the mix was removed from the thermostatic bath, it was inoculated with 1 ml of strain LPV 31, shaken, and put in a sterile Petri dish 5 cm in diameter, previously moistened with liquid vaseline to prevent the film from adhering to the glass surface.

d) Finally, the Petri dishes were placed in a vacuum drying oven at a temperature of 45°C and a pressure of 11,500 Pa for 5 h until moisture levels below 10% were reached. Another option was infrared drying for approximately 200 minutes at 50°C.

e) Once dry, the films were carefully removed from the Petri dishes to be used as dressings.

**Example 3: Viability analysis**

Once the films had been made, viability analyses were carried out to determine the bacteria's survival. The procedure was as follows:

a) A tube with 4 ml of sterile meat peptone solution at 1% was weighed.

b) Film samples with dry probiotic were taken and placed in a tube with peptone in a sterile environment.

c) The tube was left to rest for 5 min and then sonicated for 9 min in order to loosen the strain adhered to the gelatin in the medium.

d) After rehydration, the tube was shaken and 100 $\mu$l of sample were extracted and dilutions were made, 50 $\mu$l of which were placed in plates with culture medium (MRS).

e) The plates with the strain were incubated at 37°C for 24 h.

f) Finally, the colonies on the plates were counted and the results were expressed as:

$$Viability\ (cfu/g) = \frac{AVERAGE(count/dilution)}{50\mu L / 1000\ \mu L} \cdot \frac{4\ mL}{Sample\ Mass\ (g)}$$

Based on bacterial count, it was concluded that the gelatin films are an adequate vehicle for the administration of probiotic microorganisms, as they enable the survival of the latter, with a total number of microorganisms in the range of $10^6$-$10^7$ CFU/g.

**Example 4: Test treatment of burns with plasters containing strain *Lactobacillus acidophilus* LPV 31**

*4.1 Bacterial strain selected*

The strain *Lactobacillus acidophilus* LPV 31 was selected due to its probiotic properties, i.e. high hydrophobicity, hydrogen peroxide production and capacity for inhibiting the development of pathogenic strains, among others.

*4.2 Strain processing*

The probiotic strain kept in milk supplemented with yeast extract (LEL) at -20°C was activated in an MRS culture medium, using 2 subcultures at 37°C for 24 h and 14 h. For the LPV 31 strain a third inoculation was carried out in whey permeate and incubated for 14 h at 37°C in order to be finally incorporated into 2 types of support. The supports tested were plasters with different concentrations of additives.

*4.3 Animals*

Male BALB/c mice between 7 and 8 weeks of age were used. The mice were kept in cages and fed *ad libitum* under controlled temperature (25 $\pm$ 0.5°C), humidity and light conditions. At the beginning of the test the backs of the mice were shaved and they were put in cages randomly for each experimental group, with 6 animals in each group. The mice were anaesthetized when the plaster was applied and whenever it was changed, as well as being weighed during the taking of each haematological sample.

*4.4 Plaster*

Two types of plasters, designated A and B, were used. Both were formulated with 6% glycerol and 7% gelatin with 1% casein peptone. The only difference was the presence of the cross-linking agent, glutaraldehyde (0.003 M), in plaster B. In addition, for each type of plaster there was a formulation with and without incorporated bacteria. During the test, and until its completion, the plasters were changed every other day. bacterial viability was measured (CFU/ml) in the plasters at each sampling point.

*4.5 Experimental groups*

(6 mice per group):

> *Group 1:* Plaster A without bacteria (control)
> *Group 2*: Plaster B without bacteria (control)
> *Group 3*: Plaster A with strain LPV 31
> *Group 4:* Plaster B with strain LPV 31

Each mouse belonging to an experimental group was marked with an individual tail colour

*4.6 Test*

*a) Preparation of wound area:*

The hair was removed from the back of the mice and each animal was anaesthetized via a subcutaneous injection of a 0.03 ml solution of Xylazine (PACIFOR®) 2%, Ketamine (10 $\mu$g/ml) and Acepromazine 1%, in a proportion of 4:3:1.

*b) Wound:*

A 1 st to 2nd degree burn was produced on the back of each mouse with hot water (approximate temperature 90°C) for 10 seconds, affecting between 10% and 15% of the total body area. The diameter of the burn zone was 1 cm.

*c) Inoculation:*

After the wound was produced the animals were hydrated with between 0.5 and 1 ml of saline solution administered intradermally. Subsequently, the plaster was placed on the wound zone and held in place with 3M tape. Plasters were changed every other day, previous to which each animal was anaesthetized.

**d)** *Sampling:*

Blood samples were taken after 24 h (T1), 48 h (T2), 5 days (T3), 7 days (T4), 14 days (T5), 21 days (T6) and 29 days (T7). The blood samples were obtained by way of a transversal cut to each mouse's tail. Hematocrit was measured from the samples and a blood film was made for its subsequent haematological analysis. The remaining blood was subjected to immunological analysis. At each blood sampling the animal was weighed.

Tissue samples were obtained in the final sampling period, for which three animals per group, chosen randomly, were sacrificed. A ventral incision was made through which tissue from the wound zone, the liver and the spleen was aseptically removed. Each sample was macerated with 0.5 ml of PBS buffer. 50 $\mu$l of each maceration were placed in MRS agar, and the plates were incubated from 24 to 48 h at 37°C (under microaerophilic conditions). The results were expressed as CFU per gram (CFU/g) of tissue. Tissue samples were used for microbiological and histological analyses.

**Figure 7 (A1, B1, C1)** shows images of mice with five day lesions (T3). This corresponds to test group 4, which was treated with plasters containing glutaraldehyde and the strain *Lactobacillus acidophilus* LPV 31. In images **A2, B2 and C2** it is possible to observe the evolution of these lesions 29 days (T7) after the beginning of the test. The results are surprising.

**Example 5: Test treatment of burns with plasters containing strain *Lactobacillus acidophilus* LPV 31 together with pathogenic strain**

### 5.1 Bacterial strains selected

The strain used was *Lactobacillus acidophilus* LPV 31, isolated from a vaginal sample and selected for its probiotic properties, such as hydrogen peroxide production and hydrophobicity, as well as its capacity to adhere to and colonise supports.

The pathogenic strain *Pseudomonas aeruginosa* P65-00 was also used, having been isolated from a surgical wound sample.

### 5.2 Plaster

The gelatin plasters with the incorporated probiotic strain used in this test were delivered once a week. Their viability was measured each time the plasters on the animals' wounds were changed.

### 5.3 Sample processing

The probiotic strain was preserved in milk supplemented with yeast extract (LEL) at -20°C and activated in an MRS culture medium, using 2 subcultures at 37°C, for 24 h. Subsequently a third inoculation was carried out in whey permeate, incubated for 12 h and resuspended until reaching a concentration of $10^8$ CFU/ml. Finally this strain was incorporated into a plaster at a final concentration of $10^6$ CFU/ml.

The strain *Pseudomonas aeruginosa* P65-00 was preserved in glycerol 50%, activated from the culture collection in a Luria-Bertanni medium and incubated at 37°C for 18 h. Subsequently, the strain was resuspended until it reached a concentration of $10^6$ CFU/ml.

### 5.4 Animals

Male BALB/c mice between 7 and 8 weeks of age were used. The mice were kept in cages and fed *ad libitum* under controlled temperature ($25 \pm 0.5$°C), humidity and light conditions. The animals were separated into 5 experimental groups (12 mice per group):

- **-**   Group 1: Plaster (negative control)
- **-**   Group 2: Strain LPV 31 incorporated into a plaster
- **-**   Group 3: Strain LPV 31 incorporated into a plaster and strain Ps65-00
- **-**   Group 4: Ps65-00 and strain LPV 31 incorporated into a plaster
- **-**   Group 5: Ps65-00 (positive control)

### 5.5 Test

### a) Preparation of wound area:

The hair was removed from the back of the mice and each animal was anaesthetized via subcutaneous injection of a 0.03 ml solution of Xylazine (PACIFOR®) 2%, Ketamine (10 $\mu$g/ml) and Acepromazine 1%, in a proportion of 4:3:1.

### b) Wound:

The back of each mouse was burnt with water at a temperature of 90°C for 10 seconds, producing a wound covering 10% of the total body area.

### c) Plaster application and/or inoculation of pathogenic strain:

Depending on the experimental group, the plaster with or without the lactic strain and the intrahospital strain were applied superficially to the wound area.

*d) Hydration:*

The mice were hydrated with between 0.5 and 1 ml of a sterile saline solution at 0.9%, administered intradermally.

*e) Sampling times:*

3 animals per group were sacrificed after 48 h (T1), 7 days (T2) and 14 days (T3).

*f) Strain sampling and processing:*

At each sampling time, blood samples were obtained through a transversal cut to each mouse's tail. The blood was collected in a Hematocrit tube and a blood film was made for its subsequent haematological analysis. A cardiac puncture was carried out on each sacrificed animal, and the blood obtained was inoculated into selective media (MRS, Trypticase) to detect bacteremia. The samples were incubated at 37°C and analysed after 7 and 14 days.

An additional 3 animals per group were sacrificed and a ventral incision was made in each one in order to aseptically remove tissue from the wound zone, the liver and the spleen. Each sample was macerated with 0.5 ml of PBS buffer, and 50 $\mu$l of each maceration were placed in MRS or Trypticase agar. The plates were subsequently incubated from 24 to 48 h at 37°C. The cultures for isolating the lactic strain were incubated in a microaerophilic environment. In order to identify the colonies of bacteria, those cultures derived from the organ maceration samples that tested positive were analysed using microscopic analysis, oxidase tests and biochemical tests, as appropriate.

**Example 6: Clinical trial Phase 1**

*6.1 Clinical evaluation*

Local clinical reactions were evaluated for signs of induration, erythema and reddening in order to rule out allergies to the dressing or any of its components. This was done through conventional hypersensitivity tests in 5 individuals of both genders. Immediate reaction (TDD), total IgE, PCR and prick skin tests were carried out. Subsequently, each participant self-administered a 2x2 cm plaster every other day in a previously outlined area of the left forearm. The second sampling of immunological and haematological parameters was carried out 21 days after treatment administration.

None of the volunteers developed allergies. It can therefore be concluded from this experience that the probiotic components tested do not produce allergies, *in vivo* or *in vitro.*

*6.2 Clinical trial: patients with chronic ulcers*

Haematological analyses were used to evaluate the evolution of the wounds together with the application, every 48 hours, of a dressing containing the bacterial strain *Lactobacillus acidophilus* LPV 31. Microbiological aspects of the evolution of the ulcers were also evaluated (e.g. cultured pathogenic strains and molecular profiles of swabs from the wounds).

The participants were two individuals with ulcers several years old. Their results were compared with those from two participants that continued with traditional forms of treatment.

*6.3 Wound assessment and sampling*

Local evaluation of the wounds included: location, dimensions (length, width, depth), base (epithelialised, granulated, necrotic), exudate (amount, colour, odour) and characteristics of the surrounding skin. A written and photographic record was kept during the trial. Aetiological agents were identified on days 0, 5, 10, 15 and 30, using microbiological cultures. The presence or absence of *Lactobacillus sp., Pseudomonas aeruginosa, Staphylococcus aureus and Eschericia coli* was determined.

The presence of *Lactobacillus spp., Pseudomonas sp., and Staphylococcus aureus* was determined using molecular identification, specifically the polymerase chain reaction (PCR) technique.

*6.4 Haematological and cytological* analyses

Haematological and cytological studies were carried out. An initial hemogramme was carried out on the first day of treatment (day 0), and another one was carried out on the last day of the trial (day 30).

A sample of the inflammatory exudate from the wound was also obtained before the ulcer was cleaned. This sample was extended and placed on a microscope slide for subsequent analysis. This was done on days 0, 5, 10, 15 and 30, after the application of the dressing.

The effects of a gelatin dressing with the strain *Lactobacillus acidophilus* LPV-31 was assessed in an 81-year-old diabetic patient, with a chronic ulcer dating back 6 years. **Figure 9** shows the evolution of the lesion and **Table 8** the corresponding data.

**Table 8**

| DATE | 23-12-08 | 28-12-08 | 31-12-08 | 05-01-09 | 09-01-09 |
|---|---|---|---|---|---|
| SIZE | 2X2 | 2X1.5 | 1X1 | 1X1 | 1X0.5 |
| DEPTH | 0.5 | 0.5 | Superficial | Superficial | None |
| EPITHELIALISATION | - | Mild | Moderate | Yes | Scarred completely |
| GRANULATION | None | Mild | Moderate | Scarring | - |
| NECROTIC TISSUE | Yes, extensive | None | None | None | None |
| EXUDATE | - | - | - | - | - |
| AMOUNT | Abundant | Small | None | None | - |
| COLOUR | Yellow | Transparent | - | None | - |
| ODOUR | Odourless | Odourless | - | None | - |

Subsequently, the effect of a gelatin dressing with the strain *Lactobacillus acidophilus* LPV-31 was assessed in a 71-year-old volunteer suffering from chronic arterial hypertension and with an ulcer dating back 8 years. **Figure 10** shows the improvement of the lesion and **Table 9** shows the corresponding data over time.

**Table 9**

| DATE | 28-12-08 | 05-01-09 | 08-01-09 | 16-01-09 |
|---|---|---|---|---|
| SIZE | 10X12 | 10X10 | 9X9 | 9X8 |
| DEPTH | 1 cm | 0.8 | 0.5 | 0.5 |
| EPITHELIALISATION | No | Mild | Mild | Yes |
| GRANULATION | No | Yes, surroundings Yes, | Yes, surroundings | Yes |
| NECROTIC TISSUE | Yes, abundant | Wound clean | No | No |
| EXUDATE | Yes | Moderate | Moderate | Moderate |
| AMOUNT | Abundant | - | - | - |
| COLOUR | Yellow | Yellow | Yellow | Yellow |
| ODOUR | No foul odor | No foul odor | No foul odor | No foul odor |

**Claims**

1. A hydrocolloid plaster or dressing suitable for various wound types, such as infected tissue, dry wounds and deep wounds, **characterised by** the formulation of the said dressing comprising:

   a) Natural gelatin.
   b) Activated and viable lactic acid-producing strains of *Lactobacillus spp.,* preferably the strain *Lactobacillus acidophilus LPV 31.*
   c) At least one cross-linking agent, preferably glutaraldehyde.
   a) At least one plasticiser, preferably glycerol.

2. A hydrocolloid plaster or dressing, in accordance with claim 1, **characterised by** a hydration capacity of at least 2.4 kg water/kg dry film.

3. A hydrocolloid plaster or dressing, in accordance with claim 1, **characterised by** a microorganism concentration within the range of $10^6$-$10^7$ CFU/g.

4. A process for producing a hydrocolloid plaster or dressing, **characterised by** the following stages:

⚓ Activation of viable lactic acid-producing probiotic strains of *Lactobacillus spp.,* preferably *Lactobacillus acidophilus LPV 31.*

⚓ Production of reticulated gelatin films, the formula of which contains a cross-linking agent, a plasticiser and viable probiotic strains of *Lactobacillus acidophilus LPV 31,* activated in the previous stage.

⚓ An infrared or vacuum drying stage in order to achieve a moisture level below 10%.

5. A process to produce a plaster or dressing, in accordance with claim 4, **characterised by** a concentration of gelatin in a range between 1 and 10 w/w, preferably 7% w/w.

6. A process to produce a plaster or dressing, in accordance with claim 4, **characterised by** the fact that the cross-linking agent is preferably glutaraldehyde used in a concentration range between approximately 0.0005-0.003 M.

7. A process to produce a plaster or dressing, in accordance with claim 4, **characterised by** the fact that the plasticiser is preferably glycerol, used in a concentration range between approximately 1-10% v/v, preferably 6% v/v.

8. A use of a hydrocolloid plaster or dressing, **characterised by** its utilisation as a vehicle for the administration of viable probiotic microorganisms.

**Fig. Nº1**

**Fig. Nº2**

**Fig. Nº3**

A   ← GA.0M ← GA.0,001 M ← GA.0,003 M

B   ← GA.0M ← GA.0,001 M ← GA.0,003 M

**Fig. Nº4**

A   ← 35°C ← 45°C ← 55°C

B   ← 35°C ← 45°C ← 55°C

**Fig. Nº5**

A

| A: Before of drying | B: After of drying 0 M GA |
| C: After of drying 0,01 M GA | D: After of drying 0,03 M GA |

**Fig. Nº6**

**Fig. Nº 7**

**Fig. N° 8**

**Fig. N° 9**

**Fig. N° 10**

# INTERNATIONAL SEARCH REPORT

| | International application No. |
| --- | --- |
| | PCT/ CL 2010/000025 |

A. CLASSIFICATION OF SUBJECT MATTER

see extra sheet

According to International Patent Classification (IPC) or to both national classification and IPC

B. FIELDS SEARCHED

Minimum documentation searched (classification system followed by classification symbols)

A61L, A61K

Documentation searched other than minimum documentation to the extent that such documents are included in the fields searched

Electronic data base consulted during the international search (name of data base and, where practicable, search terms used)

INVENES, EPODOC, WPI, HCAPLUS, BIOSIS, GOOGLE

C. DOCUMENTS CONSIDERED TO BE RELEVANT

| Category* | Citation of document, with indication, where appropriate, of the relevant passages | Relevant to claim No. |
| --- | --- | --- |
| X | WO 2008074331 A1 (FERROSAN A/S) 26-06-2008, the whole document, specially claims 1, 24-30. | 1-5 |
| Y | CASTRO. E. et al. "Protector effect of *Lactobacillus spp.* strain in a burn of murine modthe infected by Pseudomona aeruginosa". 2008 INTERNATIONAL PROBIOTIC CONFERENCE, 4-7 of June of 2008; CONFERENCE PROCEEDINGS, page 29S, column left, abstract. | 1-5 |
| Y | WO 0061201 A1 (GANEDEN BIOTECH INC) 19.10.2000, claims. | 1-5 |
| Y | MORTAZAVIAN, A. et al. "Principles and methods of microencapsulation of probiotic microorganisms". IRANIAN JOURNAL OF BIOTECHNOLOGY. 2007. Vol. 5, Nº 1, pages 1-18. the whole document. | 1-5 |

☒ Further documents are listed in the continuation of Box C.　　　☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | |
| | "&" document member of the same patent family |

| Date of the actual completion of the international search | Date of mailing of the international search report |
| --- | --- |
| 30 September 2010　　　(30.09.2010) | **(11/10/2010)** |
| Name and mailing address of the ISA/ O.E.P.M.<br><br>Paseo de la Castellana, 75 28071 Madrid, España.<br>Facsimile No.　34 91 3495304 | Authorized officer<br>　　　M. Novoa Sanjurjo<br><br>Telephone No. +34 91 349 84 66 |

Form PCT/ISA/210 (second sheet) (July 2009)

## INTERNATIONAL SEARCH REPORT

| International application No. |
| --- |
| PCT/CL 2010/000025 |

C (continuation).                    DOCUMENTS CONSIDERED TO BE   RELEVANT

| Category* | Citation of documents, with indication, where appropriate, of the relevant passages | Relevant to claim No. |
| --- | --- | --- |
| Y | DINARVAND, R. et al. "Preparation of gelatin microspheres containing lactic acid. Effect of cross-linking on drug release". ACTA PHARMA. 01.03.2005. Vol. 55, Nº. 1, pages 57-67; abstract. | 1-5 |

Form PCT/ISA/210 (continuation of second sheet) (July 2009)

## INTERNATIONAL SEARCH REPORT

| International application No. |
| --- |
| PCT/ CL 2010/000025 |

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: 6, 7
   because they relate to subject matter not required to be searched by this Authority, namely:
   Claims 6 and 7 relate to subject matter which this Authority considers is affected by the provisions
   of PCT Rule 67.1(iv) relating to a method for treatment of the human body by therapy.
   Nevertheless, a search has been carried out for these claims on the basis of the alleged effects of
   the wound dressing containing the probiotic strains.

2. ☐ Claims Nos.:
   because they relate to parts of the international application that do not comply with the prescribed requirements to such an
   extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable
   claims.

2. ☐ As all searchable claims could be searched without effort justifying additional fees, this Authority did not invite payment of
   additional fees.

3. ☐ As only some of the required additional search fees were timely paid by the applicant, this international search report covers
   only those claims for which fees were paid, specifically claims Nos.:

4. ☐ No required additional search fees were timely paid by the applicant. Consequently, this international search report is
   restricted to the invention first mentioned in the claims; it is covered by claims Nos.:

**Remark on Protest**     ☐ The additional search fees were accompanied by the applicant's protest and, where applicable, the
payment of a protest fee.
☐ The additional search fees were accompanied by the applicant's protest but the applicable protest
fee was not paid within the time limit specified in the invitation.
☐ No protest accompanied the payment of additional search fees.

Form PCT/ISA/210 (continuation of first sheet (2)) (July 2009)

## INTERNATIONAL SEARCH REPORT

Information on patent family members

| International application No. |
| --- |
| PCT/ CL 2010/000025 |

| Patent document cited in the search report | Publication date | Patent family member(s) | Publication date |
| --- | --- | --- | --- |
| WO 2008074331 A | 26.06.2008 | CA 2673409 A | 26.06.2009 |
| | | EP 2125046 A | 02.12.2009 |
| | | CN 101641121 A | 03.02.2010 |
| | | JP 2010512865 T | 30.04.2010 |
| | | US 2010143447 A | 10.06.2010 |
| WO 0061201 A | 19.10.2000 | CA 2368509 A | 19.10.2000 |
| | | AU 4353300 A | 14.11.2000 |
| | | EP 1173233 A | 23.01.2002 |
| | | JP 2002540900 T | 03.12.2002 |
| | | US 6716435 B | 06.04.2004 |
| | | US 2004191232 A | 30.09.2004 |
| | | US 7025974 B | 11.04.2006 |
| | | AU 780367 B | 17.03.2005 |
| | | US 2006177429 A | 10.08.2006 |

Form PCT/ISA/210 (patent family annex) (July 2009)

**INTERNATIONAL SEARCH REPORT**

International application No.

PCT/ CL 2010/000025

CLASSIFICATION OF SUBJECT MATTER

*A61L 15/36* (2006.01)
*A61K 35/74* (2006.01)

**REFERENCES CITED IN THE DESCRIPTION**

**Patent documents cited in the description**

- WO 2008074331 A
- WO 2008060199 A
- US 2002131996 A
- WO 0071139 A